Europäisches Patentamt

European Patent Office

Office européen des brevets

(19)

(11) Veröffentlichungsnummer: **0 037 963**
**B1**

(12) # EUROPÄISCHE PATENTSCHRIFT

(45) Veröffentlichungstag der Patentschrift:
**07.03.84**

(51) Int. Cl.³: **A 61 K  37/02**, A 61 K  35/12

(21) Anmeldenummer: **81102458.7**

(22) Anmeldetag: **01.04.81**

(54) **Neues Protein PP9, Verfahren zu seiner Gewinnung sowie seine Verwendung.**

(30) Priorität: **10.04.80 DE 3013724**

(43) Veröffentlichungstag der Anmeldung:
**21.10.81 Patentblatt 81/42**

(45) Bekanntmachung des Hinweises auf die Patenterteilung:
**07.03.84 Patentblatt 84/10**

(84) Benannte Vertragsstaaten:
**AT BE CH DE FR GB IT LI LU NL SE**

(56) Entgegenhaltungen:
**EP - A - 0 000 134**
**FR - A - 2 364 225**
**FR - A - 2 389 637**

**CHEMICAL ABSTRACTS, Band 86, Heft 19, 9. Mai 1977, Seite 189, Zusammenfassung 135224x, COLUMBUS, OHIO (US) H. BOHN et al.: "Isolation and characterization of a new tissue protein (PP7) from human placenta"**
**CHEMICAL ABSTRACTS, Band 88, Heft 11, 13. März 1978, Seite 191, Zusammenfassung 70728j, COLUMBUS, OHIO (US) H. BOHN et al.: "Isolation and characterization of the placental protein PP5"**

(73) Patentinhaber: **BEHRINGWERKE AKTIENGESELLSCHAFT, Postfach 1140, D-3550 Marburg/Lahn (DE)**

(72) Erfinder: **Bohn, Hans, Dr., Oberer Eichweg 26, D-3550 Marburg 1 (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

## Neues Protein PP₉, Verfahren zu seiner Gewinnung sowie seine Verwendung

$PP_9$ kommt in fast allen bisher untersuchten Organen des Menschen vor. Ausser in Plazenta wurde es in folgenden fetalen Organen nachgewiesen: Herz, Leber, Niere, Lunge, Magen, Gehirn; ferner in folgenden adulten Organen: Herz, Lunge, Magen, Niere, Uterus, Leber, Milz, Nebenniere, Colon und Blase.

Aus einer menschlichen ausgewachsenen Plazenta (600 g) lassen sich mit physiologischer Salzlösung im Durchschnitt etwa 42 mg dieses Proteins extrahieren. In ähnlicher Grössenordnung dürfte die Konzentration von $PP_9$ in vielen anderen menschlichen Organen sein. Im Serum kommt $PP_9$ normalerweise nicht oder nur in Spuren ($< 0,1$ mg pro 100 ml) vor.

Gegenstand der Erfindung ist das ubiquitäre Gewebeprotein $PP_9$, gekennzeichnet durch

a) einen Gehalt an Kohlenhydraten von $5,57 \pm 1,35\%$ und davon: Hexosen $4,9 \pm 1,0\%$; Hexosamine $0,1 \pm 0,1\%$; Fucose $0,07 \pm 0,05\%$; Neuraminsäure $0,5 \pm 0,2\%$;

b) einen Sedimentationskoeffizienten $s^0_{20,w}$ von $3,2 \pm 0,2$ S;

c) ein in der Ultrazentrifuge bestimmtes Molekulargewicht von $35\,100 \pm 3800$;

d) ein im Natriumdodecylsulfat (SDS)-haltigen Polyacrylamidgel bestimmtes Molekulargewicht von $40\,000 \pm 4000$;

e) einen Extinktionskoeffizienten $E^{1\%}_{1\,cm}$ (280 nm) von $14,6 \pm 1,0$;

f) eine elektrophoretische Beweglichkeit im Bereich der $\beta_1$-Globuline;

g) einen isoelektrischen Punkt im Bereich von pH 5,0-6,8.

Zur Erläuterung der kennzeichnenden Merkmale des Gewebeproteins sei folgendes ausgeführt:

Der Sedimentationskoeffizient wurde in einer analytischen Ultrazentrifuge der Firma Beckman (Spinco-Apparatur, Modell E) bei $60\,000$ tr/min in Doppelsektorzellen mit Hilfe der UV-Scanner Technik bei 280 nm bestimmt. Als Lösungsmittel diente ein 0,05M-Phosphatpuffer (pH 6,8), der 0,2 mol/l NaCl enthielt. Die Proteinkonzentration wurde auf etwa 3 O. D. (optische Dichte) eingestellt. Die Sedimentationskoeffizienten sind auf die Basis von Wasser bei 20° C umgerechnet worden.

Zur Ermittlung des Molekulargewichtes in der UZ wurde die Sedimentationsgleichgewichtsmethode herangezogen. Die Konzentration des Proteins ist dabei auf etwa 1,0 O. D. eingestellt worden. Die Bestimmung wurde bei 9000 tr/min vorgenommen. Die Registrierung erfolgte mit UV-Optik bei 280 nm unter Einsatz des photoelektrischen Scanners.

Zur Bestimmung des Molekulargewichtes im SDS-PAA-Gel wurde ein Gel mit 7,5% Polyacrylamid (PAA), das 0,1% Natriumdodecylsulfat (SDS) enthielt, verwendet. Als Vergleichssubstanz dienten humanes Plazentalaktogen (HPL) und humanes Albumin sowie dessen Aggregate.

Zur Bestimmung des Extinktionskoeffizienten wurde die Substanz 0,10%ig in Aqua destillata gelöst.

Die Untersuchung der elektrophoretischen Beweglichkeit erfolgte in der Mikromodifikation mit dem Gerät Microzone R 200 von Beckman instruments auf Zelluloseacetatfolien (Firma Sartorius) unter Verwendung von Natriumdiäthylbarbituratpuffer, pH 8,6.

Die Bestimmung des isoelektrischen Punktes wurde mit einer Säule (440 ml) der Firma LKB, Stockholm, durchgeführt. Das sogenannte Ampholin "-Gemisch hatte bei der Untersuchung des Glykoproteins einen pH-Bereich von 5,0 bis 7,0. Das Protein $PP_9$ ist sehr heterogen. Bei der Isoelektrischen Fokussierung erscheint es im pH-Bereich von 5,0-6,8, die Hauptmenge des Proteins liegt dabei zwischen pH 6,4-6,7.

Die Bestimmung der Kohlenhydrate erfolgte nach der von H. E. Schultze, R. Schmidtberger, H. Haupt, „Biochem. Z.", 329, Seite 490 (1958), beschriebenen Methode.

Die Aminosäureanalyse wurde nach S. Moore, D. H. Spackman, W. H. Stein, „Anal. Chem.", 30, S. 1185 (1958), unter Verwendung des Flüssigkeitschromatographen Multichrom B der Firma Beckman durchgeführt.

½ Cystin wurde nach Oxydation der Proteine mit Perameisensäure (S. Moore et al. „Anal. Chem." 30, S. 1185 [1958]) und nachfolgender Chromatographie (S. Moore, „J. Biol. Chem.", 238, S. 235 [1963]) als Cysteinsäure bestimmt. Der Tryptophangehalt ist mit der direkten photometrischen Bestimmung nach H. Edelhoch, „Biochemistry", 6, S. 1948 (1967), ermittelt worden.

Die Ergebnisse der Aminosäurenanalyse des nach dem Beispiel erhältlichen $PP_9$ sind in Tabelle I zusammengestellt.

*(Tabelle auf der nächsten Seite)*

Für $PP_9$ wurden folgende Eigenschaften festgestellt, die sich zur Isolierung des neuen Gewebsproteins verwenden lassen:

1) mit Ammoniumsulfat wird $PP_9$ bei pH 7,0 zwischen 30-60% Sättigung aus wässerigen Lösungen gefällt;

2) mit wasserlöslichen Akridinbasen, z.B. 2-Äthoxi-6,9-diaminoacridinlactat (Rivanol ") wird $PP_9$ bei pH-Werten zwischen 4-9 und einer Konzentration von 0,4 bis 0,8% w/v präzipitiert, nicht oder kaum dagegen bei pH 6,0, wenn die Rivanolkonzentration $\leq 0,4\%$ beträgt;

3) bei Fällungen mit Äthanol bleibt $PP_9$ in physiologischen Salzlösungen bei pH 7,0 bis zu einer Konzentration von 25% Alkohol zur Hauptsache im Überstand;

4) bei der präparativen Elektrophorese wandert $PP_9$ im Bereich der $\beta_1$-Globuline;

5) bei der Gelfiltration (Sephadex ") erscheint $PP_9$ im Bereich der Proteine mit Molekulargewichten von $20\,000$ bis $60\,000$;

6) $PP_9$ lässt sich an schwach basische Ionenaustauscher wie z.B. DEAE-Zellulose oder DEAE-

*Tabelle I*

Aminosäurenzusammensetzung von PP$_9$
(Reste pro 100 Reste)

|  | Mol (%) | VK (%) * |
|---|---|---|
| Lysin | 8,04 | 3,49 |
| Histidin | 2,58 | 5,06 |
| Arginin | 3,49 | 1,80 |
| Asparaginsäure | 10,30 | 10,45 |
| Threonin | 4,29 | 9,82 |
| Serin | 5,70 | 10,77 |
| Glutaminsäure | 11,18 | 2,15 |
| Prolin | 6,17 | 13,61 |
| Glycin | 5,50 | 0,79 |
| Alanin | 5,69 | 1,11 |
| Cystin ½ | 2,29 | 4,38 |
| Valin | 7,12 | 2,58 |
| Methionin | 1,47 | 9,64 |
| Isoleucin | 5,28 | 5,28 |
| Leucin | 10,55 | 3,23 |
| Tyrosin | 3,95 | 9,68 |
| Phenylalanin | 3,87 | 5,48 |
| Tryptophan | 2,36 | 0,69 |

\* VK = Variationskoeffizient.

Sephadex bei niedriger Leitfähigkeit (etwa 0-2 ms) und neutral oder schwach alkalischem pH-Wert (etwa pH 7 bis 9) adsorbieren;

7) PP$_9$ kann aus seiner wässerigen Lösung durch Immunadsorption angereichert und isoliert werden.

Gegenstand der Erfindung ist deshalb ferner ein Verfahren zur Gewinnung oder Anreicherung des Gewebeproteins PP$_9$, dadurch gekennzeichnet, dass ein aus menschlichem Gewebe mittels einer Kochsalzlösung erhaltener Extrakt einer oder mehreren der folgenden Massnahmen unterworfen wird:

a) Fällung des Proteins PP$_9$ mit Ammoniumsulfat im pH-Bereich von 5 bis 8 und bei 30-60% Sättigung;

b) Fällung des Proteins PP$_9$ mit einer wasserlöslichen Acridinbase bei einem pH-Wert zwischen 4 und 9 und einer Konzentration der Base von 0,2-0,8 g/100 ml;

c) Ausfällung von Begleitproteinen aus einer Lösung von PP$_9$ in einer physiologischen Salzlösung mit Ethanol bis zu einer Konzentration von 25% bei einem pH-Wert von 5-7;

d) präparative Zonenelektrophorese und Gewinnung der $\beta_1$-Globulinfraktion;

e) Gelfiltration oder Ultrafiltration, wobei Proteine im Molekulargewichtsbereich von 20 000 bis 60 000 gewonnen werden;

f) Adsorption an einen schwach basischen Ionenaustauscher und Elution des Proteins PP$_9$;

g) immunadsorptive Anreicherung des Proteins PP$_9$;

h) Hochreinigung des Proteins PP$_9$ durch inverse Immunadsorption.

Neben Ammoniumsulfat können selbstverständlich auch andere in der präparativen Biochemie üblicherweise eingesetzten Neutralsalze zur Ausfällung des PP$_9$ verwendet werden. Neben einer Acridinbase ist auch ein wasserlösliches Derivat einer Chinolinbase, wie sie für Proteinfraktionierungen bekannt sind, einsetzbar. Entsprechend seinem elektrophoretischen Verhalten wie seinem Molekulargewicht sind zur Isolierung des Proteins auch andere Massnahmen brauchbar, die geeignet sind, ein $\beta_1$-Globulin beziehungsweise ein Protein mit Molekulargewicht um 40 000 von anderen Proteinen abzutrennen. Hierzu können die verschiedenen Methoden der Gelfiltration oder Ultrafiltration oder auch die Eigenschaft des PP$_9$ an schwach basische Ionenaustauscher gebunden und hiervon, um wieder eluiert werden zu können, verwendet werden.

Durch eine zweckmässige Kombination der genannten Massnahmen, die eine Anreicherung des PP$_9$ oder eine Abtrennung dieses Proteins von anderen Proteinen bewirken, kann das PP$_9$ isoliert werden.

Dazu können auch die chemischen oder biochemischen präparativen Äquivalente dieser Massnahmen verwendet werden.

Zum Nachweis und zur Bestimmung des PP$_9$ etwa in einer Fraktion aus einer Trennoperation können neben den angegebenen Parametern auch immunchemische Methoden dienen, da PP$_9$ antigene Eigenschaften hat.

Ein für diesen Zweck brauchbares Antiserum kann folgendermassen gewonnen werden: Durch Immunisieren von Kaninchen mit einer PP$_9$-enthaltenden Plazentaproteinfraktion (Plazentafraktionen III und V nach Bohn, H., „Arch. Gynäk." [1971], *210*, 440) wird ein polyvalentes Antiserum erhalten, mit dem PP$_9$ nachgewiesen werden kann. Dieses Antiserum kann durch Absorption mit normalem menschlichen Serum und solchen Plazentafraktionen, die PP$_9$ nicht enthalten, gegen das Antigen PP$_9$ weitgehend spezifisch gemacht werden. Dieses spezifische Antiserum kann einerseits zum immunologischen Nachweis des PP$_9$, andererseits zur Herstellung eines Immunadsorbens dienen, das zur Anreicherung und Isolierung von PP$_9$ eingesetzt werden kann.

Zum immunologischen Nachweis von PP$_9$ kann die Geldiffusionstechnik nach Ouchterlony (vgl. Schultze und Heremans, „Molecular Biology of Human Proteins", vol. 1, S. 134) herangezogen werden.

Mit Hilfe des nach der vorliegenden Anmeldung gewonnenen PP$_9$ lassen sich durch Immunisieren von Tieren nach bekannten Methoden monospezifische Antiseren herstellen.

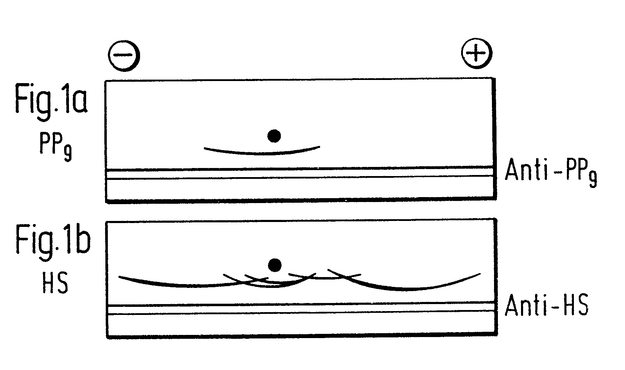

PP$_9$ hat antigene Eigenschaften. Bei der Immunisierung von Tieren mit diesem Protein werden spezifische Antikörper gebildet. Die immunologische Reaktion von PP$_9$ mit einem spezifischen Antiserum vom Kaninchen nach Auftrennung im elektrischen Feld in Agar haltigem Gel zeigt Abbildung 1 a. Abbildung 1 b bringt zum Vergleich dazu die Auftrennung der Proteine des Serums, sichtbar gemacht durch deren Immunreaktion mit einem Antiserum vom Kaninchen gegen Humanserum (HS).

Der Nachweis und die Bestimmung von $PP_9$ mit immunologischen Methoden haben diagnostische Bedeutung. $PP_9$ ist ein offensichtlich in fast allen menschlichen Organen vorkommendes Gewebeprotein. Bei Erkrankungen, die mit Gewebezerfall einhergehen, ist dieses Protein in erhöhter Konzentration im Blut nachweisbar. Seine Bestimmung kann daher ganz allgemein zur Erkennung von Erkrankungen sowie zur Überwachung des Krankheitsverlaufs und zur Kontrolle der Therapie verwendet werden.

$PP_9$ kann also verwendet werden, um Antiseren herzustellen, die dazu dienen können, $PP_9$ nachzuweisen und zu bestimmen.

Die Erfindung wird am nachstehenden Beispiel erläutert:

*Beispiel*

A) *Extraktion der Plazenten und Fraktionierung des Extraktes mit Rivanol und Ammoniumsulfat*

1000 kg tiefgefrorene menschliche Plazenten werden im Schneidmischer zerkleinert und mit 1000 l einer 0,4%igen Kochsalzlösung extrahiert. Der Extrakt wird dann, nach Abtrennung des Geweberückstandes durch Zentrifugation, mit 20%iger Essigsäure auf pH 6,0 eingestellt und unter Rühren mit 200 l einer 3%igen Lösung von 2-Äthoxy-6,9-Diaminoacridinlactat (Rivanol[®], Hoechst AG) versetzt. Der entstehende Niederschlag wird abzentrifugiert und verworfen. Zum Überstand gibt man 1% m/v Bentonit A (Firma Erbslöh & Co., Geisenheim/Rhein), stellt den pH-Wert durch Zugabe von 2N-NaOH auf 7,0 ein und filtriert. Das Filtrat wird unter Rühren langsam mit 30% m/v Ammoniumsulfat versetzt; das Plazentaprotein $PP_9$ fällt dabei, zusammen mit anderen Proteinen, aus. Man filtriert den Niederschlag ab und erhält etwa 12 kg einer feuchten Paste, die im nachfolgenden als Fraktion A bezeichnet wird. 500 g dieser Paste enthalten im Durchschnitt etwa 640 mg $PP_9$.

B) *Gelfiltration an Sephadex G-150*

500 g der Fraktion A werden in etwa 400 ml Wasser gelöst und gegen einen 0,1 M-Tris-HCl-puffer (pH 8,0), der 1,0 mol/l NaCl und 0,1% $NaN_3$ enthält (Pufferlösung I) dialysiert.

Die proteinhaltige Lösung wird auf eine mit Sephadex G-150 gefüllte Säule (10×100 cm) aufgetragen und gelfiltriert. Zum Eluieren wird die Pufferlösung I verwendet. Die Eluate testet man im Geldiffusionstest nach Ouchterlony mit einem spezifischen anti-$PP_9$-Kaninchenserum; alle das plazentaspezifische Protein $PP_9$ enthaltenden Fraktionen werden gesammelt und auf einem Ultrafilter (Amicon UF 2000) unter Verwendung von PM-10-Membranen auf etwa 300 ml eingeengt. Diese Lösung (Fraktion B) enthält insgesamt etwa 600 mg $PP_9$.

C) *Anreicherung von $PP_9$ durch Immunsadsorption*

1. *Herstellung des Immunadsorbens.*
350 ml eines anti-$PP_9$-Serums vom Kaninchen werden gegen 0,02M-Phosphatpuffer (pH 7,0) dialysiert und zur Abtrennung der Immunglobuline an DEAE-Zellulose chromatographiert. Die Immunglobulinfraktion (2,78 g Protein) wird dann mit 278 g besonders gereinigter Agarose in Kugelform (Sepharose[®] 4 B der Pharmacia, Uppsala, Schweden), die mit 348 g Bromcyan aktiviert worden war, umgesetzt und so kovalent an einen Träger gebunden.

Das Verfahren ist beschrieben von Axen R., Porath, J., Ernbach, S., „Nature", *214*, S. 1302 (1967).

Mit Hilfe eines auf diese Weise hergestellten Immunadsorbens kann das Plazentaprotein $PP_9$ aus dessen Lösung, insbesondere aus $PP_9$-angereicherten Plazentaextraktfraktionen isoliert werden.
2. *Durchführung der Immunadsorption.*

Das Immunadsorbens wird in Pufferlösung I (0,1 M-Tris-HCl-puffer, pH 8,0 mit 1,0 mol/l NaCl und 0,1% $NaN_3$) suspendiert, dann in eine Chromatographiesäule gefüllt (5,5×20 cm) und mit Pufferlösung I nachgespült. Dann lässt man langsam 60 ml der $PP_9$-haltigen Lösung (Fraktion B) durch die Säule wandern, wobei $PP_9$ immunadsorptiv gebunden wird. Man wäscht die Säule gründlich mit Puffer I nach und eluiert dann das adsorbierte Protein mit etwa 600 ml 3M-Kaliumrhodanidlösung. Die $PP_9$-haltigen Eluate werden gegen Pufferlösung I dialysiert und im Ultrafilter auf ca. 15 ml eingeengt.

Ausbeute pro Adsorption: ~ 10 mg $PP_9$.

Das Adsorbens in der Säule wird unmittelbar nach der Elution von $PP_9$ wieder mit Pufferlösung I neutralisiert und gründlich gewaschen; es kann dann erneut zur (immun-)adsorptiven Bindung von $PP_9$ eingesetzt werden.

D) *Hochreinigung von $PP_9$*

Das durch Immunadsorption gewonnene Protein ist häufig durch unspezifisch gebundene Serumproteine und andere Plazentagewebeproteine verunreinigt. Die Abtrennung der Hauptmenge der Serumbegleitproteine gelingt z.B. durch Gelfiltration an Sephadex G-150. Die restlichen Begleitproteine werden dann durch inverse oder negative Immunadsorption, d.h. mit Hilfe von trägergebundenen Antikörpern gegen die noch als Verunreinigung vorliegenden Proteine, entfernt.

So wurden die in geringer Menge noch vorliegenden Proteine $SP_1$ (Bohn, H. *et al.*, „Blut" [1976], *32*, 103), $PP_7$ (Bohn, H. und Winckler, W., „Arch. Gynäk." [1977], *222*, 5) und HPG-2 (Bohn, H. und Winckler, W., „Blut" [1977], *25*, 305) durch entsprechende Immunadsorbentien entfernt. Ausser diesen bereits bekannten Proteinen wurden aber in der $PP_9$-Rohfraktion noch einige andere, bisher unbekannte Gewebeproteine der Plazenta nachgewiesen. Zur Herstellung von Antikörpern gegen diese Begleitproteine wurde die $PP_9$-Rohfraktion 12 h gegen einen 0,5M-Glycin-HCl-puffer (pH 2,5) dialysiert und dann wieder neutralisiert. Unter diesen Bedingungen wird $PP_9$ denaturiert und verliert seine immunchemische Reaktivität; die Verunreinigungen dagegen sind stabil und bleiben als Antigen erhalten.

7     **0 037 963**     8

Immunisiert man damit Tiere, so erhält man Antikörper gegen diese Proteine, die nach Bindung an einen Träger zur immunadsorptiven Entfernung der unbekannten Plazentaproteine aus der $PP_9$-Rohfraktion eingesetzt werden können.

**Patentansprüche**

1. Protein $PP_9$, gekennzeichnet durch

a) einen Gehalt an Kohlenhydraten von $5,57 \pm 1,35\%$ und davon: Hexosen $4,9 \pm 1,0\%$; Hexosamine $0,1 \pm 0,1\%$; Fucose $0,07 \pm 0,05\%$; Neuraminsäure $0,5 \pm 0,2\%$;

b) einen Sedimentationskoeffizienten $s_{20,w}^0$ von $3,2 \pm 0,2$ S;

c) ein in der Ultrazentrifuge bestimmtes Molekulargewicht von $35\,100 \pm 3800$;

d) ein im Natriumdodecylsulfat (SDS) -haltigen Polyacrylamidgel bestimmtes Molekulargewicht von $40\,000 \pm 4000$;

e) einen Extinktionskoeffizienten $E_{1\,cm}^{1\%}$ (280 nm) von $14,6 \pm 1,0$;

f) eine elektrophoretische Beweglichkeit im Bereich der $\beta_1$-Globuline;

g) einen isoelektrischen Punkt im Bereich von pH 5,0-6,8.

2. Verfahren zur Gewinnung oder Anreicherung des Gewebeproteins $PP_9$ nach Anspruch 1, dadurch gekennzeichnet, dass ein aus menschlichem Gewebe mittels einer Kochsalzlösung erhaltener Extrakt einer oder mehreren der folgenden Massnahmen unterworfen wird:

a) Fällung des Proteins $PP_9$ mit Ammoniumsulfat im pH-Bereich von 5 bis 8 und bei 30-60% Sättigung;

b) Fällung des Proteins $PP_9$ mit einer wasserlöslichen Acridinbase bei einem pH-Wert zwischen 4 und 9 und einer Konzentration der Base von 0,2-0,8 g/100 ml;

c) Ausfällung von Begleitproteinen aus einer Lösung von $PP_9$ in einer Physiologischen Salzlösung durch Zugabe von Ethanol bis zu einer Konzentration von 25% bei einem pH-Wert von 5-7;

d) präparative Zonenelektrophorese und Gewinnung der $\beta_1$-Globulinfraktion;

e) Gelfiltration oder Ultrafiltration, wobei Proteine im Molekulargewichtsbereich von 20 000 bis 60 000 gewonnen werden;

f) Adsorption an einen schwach basischen Ionenaustauscher und elution des Proteins $PP_9$;

g) immunadsorptive Anreicherung des Proteins $PP_9$;

h) Hochreinigung des Proteins $PP_9$ durch inverse Immunadsorption.

3. Verwendung des Proteins nach Anspruch 1 zur Gewinnung eines Antiserums zum immunologischen Nachweis und zur Bestimmung dieses Proteins.

**Revendications**

1. Protéine $PP_9$, caractérisée par:

a) une teneur en hydrates de carbone de $5,57 \pm 1,35\%$, dans lesquels: hexoses $4,9 \pm 1,0\%$; hexosamine $0,1 \pm 0,1\%$; fucose $0,07 \pm 0,05\%$; acide neuraminique $0,5 \pm 0,2\%$;

b) une constante de sédimentation $S_{20,w}^0$ de $3,2 \pm 0,2$ S;

c) un poids moléculaire déterminé par ultracentrifugation de $35\,100 \pm 3800$,

d) un poids moléculaire déterminé dans un gel de polyacrylamine contenant du dodécylsulfate de sodium (DSS) de $40\,000 \pm 4000$;

e) un coefficient d'extinction $E_{1\,cm}^{1\%}$ (280 nm) de $14,6 \pm 1,0$;

f) une mobilité électrophorétique dans le domaine des $\beta_1$-globulines, et

g) un point isoélectrique dans le domaine de pH de 5,0-6,8.

2. Procédé de préparation ou de concentration de la protéine tissulaire $PP_9$ suivant la revendication 1, caractérisé en ce qu'un extrait obtenu à partir de tissu humain au moyen d'une solution de chlorure de sodium est soumise à une ou plusieurs des mesures suivantes:

a) précipitation de la protéine $PP_9$ avec du sulfate d'ammonium dans le domaine de pH de 5 à 8 et à 30-60% de la saturation;

b) précipitation de la protéine $PP_9$ avec une base acridinique soluble dans l'eau à un pH entre 4 et 9 et à une concentration de la base de 0,2-0,8 g/100 ml;

c) précipitation d'impuretés protéiniques d'une solution de $PP_9$ dans une solution saline physiologique d'éthanol jusqu'à une concentration de 25% à un pH de 5-7;

d) électrophorèse de zone préparative et préparation de la fraction $\beta_1$-globulinique;

e) filtration sur gel, ou ultrafiltration, dans laquelle on obtient des protéines dans le domaine de poids moléculaires de 20 000 à 60 000;

f) adsorption sur un échangeur d'ions faiblement basique et élution de la protéine $PP_9$;

g) concentration par immuno-adsorption de la protéine $PP_9$;

h) purification poussée de la protéine $PP_9$ par immuno-adsorption inverse.

3. Utilisation de la protéine selon la revendication 1 pour la préparation d'un antisérum pour la détection immunologique et le dosage de cette protéine.

**Claims**

1. Protein $PP_9$, characterized by

a) a content of carbohydrates of $5.57 \pm 1.35\%$, of which are $4.9 \pm 1.0\%$ hexoses, $0.1 \pm 0.1\%$ hexoseamines, $0.07 \pm 0.05\%$ fucose, $0.5 \pm 0.20$ neuraminic acid,

b) a sedimentation coefficient $s_{20,w}$ of $3.2 \pm 0.2$ S,

c) a molecular weight of $35,100 \pm 3,800$ determined in a ultracentrifuge,

d) a molecular weight of $40,000 \pm 4,000$ determined in polyacrylamide gel containing sodium dodecylsulfate (SDS),

e) an extinction coefficient $E_{1\,cm}^{1\%}$ (280 nm) of 14.6±1.0,

f) an electrophoretic mobility in the range of the $\beta_1$-globulins, and

g) an isoelectric point in the pH range of 5.0-6.8.

2. Process for preparing or enriching the tissue protein according to claim 1, which comprises subjecting an extract obtained from human tissue by means of a saline solution to one or more of the following measures:

a) precipitating the protein $PP_9$ with ammonium sulfate in the pH-range of from 5 to 8 and at a saturation of 30 to 60%;

b) precipitating the protein with a watersoluble acridine base at a pH-value of between 4 and 9 and a concentration of the base of from 0.2 to 0.8 g/100 ml;

c) precipitating accompanying proteins out of a solution of $PP_9$ in a physiologic salt solution by adding ethanol up to a concentration of 25% at a pH of 5-7,

d) preparative zone electrophoresis and isolating the $\beta_1$-globulin fraction;

e) gel-filtration or ultrafiltration, whereby proteins in the molecular weight range of from 20,000 to 60,000 are isolated;

f) adsorbing $PP_9$ on a weakly basic ion exchanger and eluting it;

g) immuno-adsorptive enrichment of $PP_9$;

h) ultra-purification of $PP_9$ by inverse immuno-adsorption.

3. Use of the protein according to claim 1 for preparing an antiserum useful in the immunologic detection and determination of this protein.

Fig.1a
PP_g                                    Anti-PP_g

Fig.1b
HS                                      Anti-HS